(19) Europäisches Patentamt
European Patent Office
Office européen des brevets

(11) Publication number: **0 429 816 A1**

(12) **EUROPEAN PATENT APPLICATION**

(21) Application number: **90119582.6**

(22) Date of filing: **12.10.90**

(51) Int. Cl.[5]: **C07K 17/02**, A61K 39/385

(30) Priority: **31.10.89 GB 8924438**

(43) Date of publication of application: **05.06.91 Bulletin 91/23**

(84) Designated Contracting States: **AT BE CH DE DK FR GB IT LI LU NL SE Bulletin**

(71) Applicant: **F. HOFFMANN-LA ROCHE AG**
**Postfach 3255**
**CH-4002 Basel(CH)**

(72) Inventor: **Etlinger, Howard, Dr.**
**4 Untertalweg**
**CH-4144 Arlesheim(CH)**

(74) Representative: **Mezger, Wolfgang, Dr. et al**
**Grenzacherstrasse 124 Postfach 3255**
**CH-4002 Basel(CH)**

# (54) Vaccine composition with non-immunosuppresive T-cell epitope component.

(57) The present invention provides novel compositions which are useful as antigens in vaccines for eliciting a strong protective immune response against a pathogenic agent in a host. The said compositions comprise a compound representing a B cell epitope which is an antigen from a pathogenic agent or an antigenic sub-part thereof and a compound representing a T helper cell epitope which is a sub-part of an antigen from a pathogenic agent characterized in that the compound representing the T helper cell epitope contains information for carrier function but not for epitope suppressive function. The invention also relates to processes for the preparation of the said compositions, to vaccines comprising the same and to the use of these compositions and vaccines for providing protective immunity against a pathogenic agent. Furthermore the present invention relates to methods for eliciting a protective immune response against a pathogenic agent in a host and to methods for determining whether a compound representing the T helper cell epitope contains information for carrier function but not for epitope suppressive function.

EP 0 429 816 A1

## VACCINE COMPOSITION WITH NON-IMMUNOSUPPRESSIVE T-CELL EPITOPE COMPONENT

The present invention relates to novel compositions for eliciting a strong protective immune response against a pathogenic agent in a host and to vaccines containing the said compositions.

It is known that certain antigens require the co-operation of T helper cells and B cells for the generation of a humoral immune response. Such antigens comprise T cell epitopes and B cell epitopes.

T helper cell epitopes are sub-parts of antigens such as proteins or complex carbohydrates which sub-parts can be presented by an antigen presenting cell (APC) to T helper cells which in turn are then activated and "help" B cells to produce specific antibodies directed against the antigen in question in a helper T cell dependent immune response (Schwartz, Ann. Rev. Immunol. 3, 237-261 [1985]). A large number of T cell epitopes are known to the person skilled in the art (see for example Good et al., Proc. Natl. Acad. Sci. USA 85, 1199-1203 [1988] and Francis et al., Nature 330, 168-170 [1987]). T helper cell epitopes may be predicted using the methods of DeLisi et al., Proc. Natl. Acad. Sci. USA 82, 7048-7052 [1985] and Rothbard et al., EMBO J. 7, 93-100 [1988].

The strict genetic control of responsiveness to individual T cell epitopes by the polymorphic class II MHC genes limits the usefulness of vaccines comprising single T cell epitopes. However it has been found recently that certain T-cell epitopes are recognized by a large number of DR haplotypes and thus can be used as universal T-cell epitopes (Sinigaglia et al., Nature 336, 778-780 [1988]).

B cell epitopes are regions of an antigen such as a peptide, a polypeptide, a hapten or a carbohydrate which regions can be recognized by antibodies. It has been found that synthetic sub-parts of an antigen forming a B cell epitope are capable of inducing a protective immune response in a host. Thus, for example, chemically synthesized peptides representing a B-cell epitope are capable to induce the formation of antibodies which bind to the native molecule in a host (Arnon et al., Proc. Natl. Acad. Sci. USA 68, 1450-1455 [1971]). It has to be noted that in order for such a peptide which represents a B cell epitope to elicit an immune response in a host the peptide has to be bound to a carrier. Such carriers are usually proteins. A large number of such carrier proteins are known to the person skilled in the art. The carrier protein may also provide the T cell epitope function mentioned above.

Some proteins which are currently used in vaccines as carrier protein for single B cell epitopes are themselves used as antigens in vaccines. Examples for proteins used as carrier proteins in vaccines are the tetanus toxin of Clostridium tetani which is the pathogenic agent causing tetanus and the diphtheria toxin of Corynebacterium diphtheriae (or Klebs-Loeffler bacillus) which is the pathogenic agent causing diphtheria. It has to be noted that these toxins are used as carrier protein in the toxoid form, i.e. the detoxified form.

The following Table I, which is not meant to be complete, lists a few pathogenic agents against which vaccines are currently available or will be available in the foreseeable future and wherefrom the T cell epitopes and/or the B cell epitopes of the present invention may be derived:

Table I

| population | pathogenic agent | disease |
|---|---|---|
| human | Corynebacterium diphtheriae (Klebs-Loefller bacillus) | diphtheria |
| | Streptococcus pneumoniae | pneumonia |
| | Clostridium tetani | tetanus |
| | Bacillus Calmette-Guérin | tuberculosis |
| | Bordetella pertussis | pertussis |
| | Vibrio cholerae | cholera |
| | mumps virus | mumps |
| | measles virus | measles |
| | rubella virus | rubella |
| | adenovirus | respiratory d. |
| | polio virus | polio |
| | Bacillus anthracis | anthrax |
| | rota virus | diarrhoea |
| | hepatitus virus | hepatitis |
| | rabies virus | rabies |
| | HIV-1 | AIDS |
| | HIV-2 | AIDS |
| | HTLV | leukemia |
| | influenza virus | influenza |
| | pox virus | small pox |
| | Yersinia pestis | plague |
| | Plasmodium falciparum | malaria |
| cows | foot-and-mouth disease virus | foot-and-mouth disease |
| chicken | Eimeria tenella | coccidiosis |
| | Eimeria acervulina | coccidiosis |

The vaccines directed against the pathogenic agents mentioned above may be used for providing protective immunity to substantially all members of a population. Due to variable individual responsiveness to vaccination it is almost impossible to provide protective immunity to all members of a population. However, since pathogenic agents are usually dependent on a close interaction between carriers of the pathogenic agent and non-immune hosts for causing an infection, the presence of a low number of non-immune individuals in a given population usually does not jeopardize the success of a vaccination program.

As indicated above proteins from the pathogenic agents listed in Table I may be used as carriers for sub-unit vaccines representing B cell epitopes. Examples of proteins which are currently used in vaccines for providing protective immunity to substantially all members of a population and which have been employed in newly developed vaccines as carriers for such B cell epitopes are the diphtheria toxin from Corynebacterium diphtheriae and the tetanus toxoid from Clostridium tetani. Herrington et al., Nature 328, 257-259 [1987] have conjugated the 12 amino acid synthetic peptide $(NANP)_3$ comprising the immunodominant B cell epitope of Plasmodium falciparum circumsporozoite (CS) protein to the tetanus toxoid (TT). The composition obtained in this way was adjuvanted with aluminium hydroxyde and administered intramuscularly in three doses at monthly intervals to 35 healthy human volunteers as a malaria vaccine. It was found that this malaria vaccine lead to a protective immune response in only a few individuals. It was proposed that tetanus toxoid given to the volunteers in the past as immunizations dampened the immune response to the synthetic peptide component (B-cell epitope) of the conjugate.

In the meantime it has become evident that this observation can be generalized. It is known that an animal which is injected with an antigen such as a protein responds to a second injection with the antigen by producing a markedly enhanced antibody response. If the second injection is made with the original antigen which has been modified, for example by the addition of a hapten or peptide, the response to the antigen is similarly enhanced but the response to the peptide or hapten is typically inhibited. This inhibition, which is termed "epitope suppression", is mediated by T and B cells.

Epitope suppression is thought to be caused by the presence of B cells which react with epitopes of the original antigen. These B cells which have been expanded as a result of the initial injection with the said original antigen are able to out-compete the B cells directed to the modified portions of the antigen thus

leading to a reduced immune response against the said modified portions (Schutze et al., Cell. Immunol. 104, 79-90 [1987]). In addition, T suppressor cells which appear to be reactive with the unmodified portions of the antigen have been shown to play a role in the inhibition of the antibody response to the B-cell epitopes represented by the said modified portions of the antigen (Tagawa et al., Cell. Immunol. 86, 327-336 [1984]). In the poor immune response to $(NANP)_3$ observed by Herrington et al. (supra) the original antigen would be represented by the carrier protein, i.e. TT, and the modified portions would be represented by the compound representing the B-cell epitope, i.e. the $(NANP)_3$ determinants.

Sub-unit vaccines consisting of sub-parts of antigens are a safe alternative to vaccines containing inactivated or attenuated pathogenic agents especially in cases where the residual virulence of the pathogenic agent is not negligible. Thus on the one hand sub-unit vaccines are desirable for the reasons mentioned above and on the other hand it is known that epitope suppression may lead to an ineffective single B cell epitope vaccine (Herrington et al., supra). Therefore a new approach for designing sub-unit vaccines had to devised.

It is known that sequences recognized by helper T cells and suppressor T cells can be distinguished (Adorini et al., J. Exp. Med., 150, 293-306 [1979]). Therefore it was proposed that in a given antigen regions or sub-parts representing T cell epitopes which contain information for carrier function but not epitope suppressive function are distinguishable. A new test system had to be devised which test systems allowed the above distinction. Using this test system it has now been found in the present invention that the information for carrier function can be separated from that for the epitope suppressive function in an antigen. Thus the present invention made possible the preparation of vaccines comprising as T helper cell component a compound representing a T cell epitope containing information for carrier function but not epitope suppressive function.

Therefore the present invention provides compositions comprising a compound representing a B cell epitope which is an antigen from a pathogenic agent or an antigenic sub-part thereof and a compound representing a T helper cell epitope which is a sub-part of an antigen from a pathogenic agent characterized in that the compound representing the T helper cell epitope contains information for carrier function but not for epitope suppressive function. The said compounds may originate from the same or from different pathogenic agents. They may also originate from the same protein. In the latter case it would be necessary to remove sequences which contain information for suppressive function or alternatively to alter these sequences in such away that they do no longer shcw suppressive function. The present invention also relates to the processes for the preparation of these compositions, to vaccines comprising the same and to the use of these compositions and vaccines for providing protective immunity against a pathogenic agent. The said composition may be a mixture of the individual compounds mentioned above or may be one single chemical unit.

A compound representing a B cell epitope is defined herein as being a peptide, a polypeptide, a hapten or a carbohydrate derived from a pathogenic agent such as a disease-causing bacterium, virus, fungus or parasite. Examples of such pathogenic agents are listed in Table I and described in Davis et al., "Microbiology", 3rd ed., Harper International Edition. Preferred compounds representing a B cell epitope are peptides and polypeptides from such pathogenic agents. The advent of recombinant DNA technology has made possible to determine the nucleotide sequence and the amino acid sequence of a large number of antigens from pathogenic agents. The known amino acid sequence of these antigens has made possible the generation of synthetic antigens such as recombinant polypeptides and synthetic peptides. A large number of such synthetic antigens are known (see: "Vaccines 89: Modern approaches to new vaccines including prevention of AIDS", R.A. Lerner, H. Ginsberg, R.M. Chanock and F. Brown eds., Cold Spring Harbor Laboratory [1989], Cold Spring Harbor, New York, U.S.A.). Preferred compounds representing B cell epitopes are sub-parts of antigens from a malaria parasite. An example for such an antigen is the circumsporozoite (CS) protein. Most preferably the compound representing a B cell epitope is a peptide comprising the sequence $(NANP)_n$ of the repeat region of the CS protein wherein n is about 3 to 50.

The compound representing a T helper cell epitope is a sub-part of an antigen such as a protein or complex carbohydrate which sub-part representing a T helper cell epitope may be defined using the method of De Lisi et al. (supra) and/or Rothbard et al. (supra). Whether the said compound representing a T helper cell epitope provides carrier function but does not provide an epitope suppressive function can be determined using the following test system which is also part of the present invention:

(a) Antibodies directed against the antigen wherefrom the compound representing the T helper cell epitope is a sub-part thereof are tested for reactivity with the compound representing the said putative T helper cell epitope. These antibodies should not react with the said compound representing the putative T helper cell epitope.

(b) The compound representing the putative T helper cell epitope defined in step (a) is then linked to a

compound representing a B cell epitope. The composition formed in this way (termed composition A) is then tested for its activity to induce the formation of antibodies in non-immunized hosts and in hosts which have previously been injected with the antigen wherefrom the compound representing the T helper cell epitope is a sub-part thereof. These activities are then compared with the activities of a composition comprising a compound representing the same B cell epitope linked to the above-mentioned antigen (termed composition B) in non-immunized hosts and in hosts which have previously been injected with the above-mentioned antigen.

The compound representing the T helper cell epitope contains information for carrier function but not for epitope suppressive function when composition A and composition B

(I) have about equal capability to induce the formation of antibodies in non-immunized hosts; and

(II) an enhancement of the antibody response is observed with composition A in hosts which have previously been injected (preimmunized) with the above-mentioned antigen in comparison to composition B wherein the said response is suppressed.

As outlined above the injection of a composition comprising an antigen as a carrier for a compound representing a B cell epitope into a host which has previously been injected with the said antigen leads to a reduced antibody response due to epitope suppression. It has now been observed that with the present invention the potential disadvantage of using an antigen which has already been used in previous immunizations as a carrier for sub-unit vaccines can be converted to an advantage by using a portion of the original antigen as a carrier which portion does not contain information for an epitope suppressive function. Thus for example it has been found that when a composition of the present invention comprising a compound representing a T cell epitope which compound is a sub-part from an antigen against which the host is preimmunized and which compound has information for carrier function but not for epitope suppressive function is used to vaccinate the host, the first injection of the said composition or a vaccine comprising it is taken by the immune system like a secondary injection. This is due to the T helper cell priming caused by the preimmunization. The first injection of a vaccine comprising a compound of the present invention in a preimmunized host is therefore strongly enhanced in comparison to such a first injection in a host without T helper cell priming due to preimmunization with the antigen wherefrom the compound representing the T helper cell epitope in the composition is a sub-part thereof. This enhanced immune response after a primary injection is especially important in cases where a high titer of protective antibodies has to be obtained quickly to counteract the effects of a virulent pathogenic agent to which an individual will be exposed shortly after vaccination.

Thus the present invention also relates to a method for eliciting a protective immune response against a pathogenic agent, which method comprises stimulating the immune system of a host with an immunizing amount of a composition or a vaccine of the present invention, preferably to a method wherein the host is preimmunized with an antigen which is the antigen wherefrom the compound representing the T helper cell epitope in the composition of the present invention is a sub-part thereof. The time interval selected between the preimmunizations and the immunizations is not critical and can vary between a few days, e.g. at least about 5 to 10 days, to many weeks, months or even years. A person skilled in the art is in a position to select appropriate time intervals or to design immunization protocols in accordance with the present invention which are optimized in regard to these time intervals.

Furthermore it has also been found that when a host, which had been treated with the method for eliciting a protective immune response mentioned above, is again immunized with a composition or a vaccine of the present invention, but which composition comprises a compound representing a B cell epitope which is different from the one present in the earlier immunizations, then an enhanced immune response is obtained against the said new B cell epitope. Preferably this new B cell epitope is derived from a different host than the host wherefrom the B cell epitope used for the previous immunizations was derived.

Therefore the present invention also relates to a method for eliciting a protective immune response against a pathogenic agent $P_0$ in a host (e.g. a human or an animal), which host has been preimmunized:

(a) first with an antigen from a pathogenic agent $P_1$ and then at least once, preferably once or twice, with a composition C comprising a compound representing a B cell epitope which is an antigen from the pathogenic agent $P_2$ or an antigenic sub-part thereof and a compound representing a T helper cell epitope which is a sub-part of an antigen from the pathogenic agent $P_1$, characterized in that the compound representing the T helper cell epitope contains information for carrier function but not for an epitope suppressive function or

(b) at least once, preferably twice with the composition C mentioned above,

which method comprises stimulating the immune system of the host with an immunizing amount of a composition C′ comprising a compound representing a B cell epitope which is an antigen from the

pathogenic agent $P_0$ or an antigenic sub-part thereof and a compound representing a T helper cell epitope which is a sub-part of an antigen from the pathogenic agent $P_1$ characterized in that the compound representing the T helper cell epitope contains information for carrier function but not for an epitope suppressive function.

An enhanced immune response against the pathogenic agent $P_0$ is the result of the above immunization method. This finding is surprising since the person skilled in the art would have expected that the preimmunizations mentioned above would rather lead to an inhibition of the immune response. The surprising finding means that as new protective B cell epitopes are identified, hosts which have been preimmunized according to (a) or (b) as indicated above, will respond to an immunization with a composition of the present invention comprising a compound representing the said new protective B cell epitope with an enhanced immune response.

Examples of antigens which are widely used in vaccination programs and thus could be used as sources for T helper sequences having information for carrier function but not epitope suppressive function in the preimmunizations mentioned above are antigens derived from the pathogenic agents selected from the group of pathogenic agents listed in Table 1 such as Corynebacterium diphtheriae (Klebs-Loeffler bacillus), Clostridium tetani, Bordella pertussis, Vibrio cholerae, mumps virus, measles virus, rubella virus, polio virus, rota virus, hepatitis virus, influenza virus and pox virus et al.

Thus the present invention preferably relates to compositions wherein the compound representing a T-cell epitope is a sub-part of an antigen or is derived from an antigen from a pathogenic agent mentioned above. More preferably the said compound is a sub-part of the tetanus toxin or the diphtheria toxin, most preferably the sub-part of the tetanus toxin which is the polypeptide TT73-99 having the amino acid sequence

Tyr-Asp-Pro-Asn-Tyr-Leu-Arg-Thr-Asp-Ser-Asp-Lys-Asp-Arg-Phe-Leu-Gln-Thr-Met-Val-Lys-Leu-Phe-Asn-Arg-Ile-Lys (I)

or an equivalent thereof.

An equivalent of the polypeptide mentioned above is defined as being a polypeptide with an amino acid sequence which differs from the amino acid sequence (I) by deletions, insertions and/or amino acid substitutions. Examples of such equivalent polypeptides are the polypeptides having an amino acid sequence corresponding to the amino acid sequence (I) but wherein up to about fiveteen, preferably only 1, 2, 3, 4 or 5 amino acids at the N-terminus and/or the C-terminus are deleted such as the polypeptide TT88-99 having the amino acid sequence

Leu-Gln-Thr-Met-Val-Lys-Leu-Phe-Asn-Arg-Ile-Lys (II)

or an equivalent thereof.

Amino acid substitutions which potentially do not alter the secondary or tertiary structure of peptides are known from the article by R.F. Doolittle in "The Proteins", Vol. IV, Neurath, H. and Hill R.L., Eds., Academic Press, New York, p. 1-119, [1979].

It has to be noted that an antigenic determinant in a peptide or polypeptide generally comprises at least 6 amino acid residues and thus the peptides i.e. the compounds mentioned above should have at least about this size. It is understood that in cases where the said compound is large, e.g. is in the form of a peptide which comprises more than 6 amino acid residues, the compound may represent in fact more than one epitope, whereby these epitopes may also overlap. Therefore wherever it is refered to a single epitope in the present case a plurality of epitopes is meant to be encompassed.

The peptide or polypeptide representing a B cell epitope or a T cell epitope may be prepared using conventional peptide synthetic methods, either in solution or, preferably by the solid phase method of Merrifield (J. Am. Chem. Soc. 85, 2149-2154 [1963]) or by any other equivalent methods known in the art.

Solid phase synthesis is commenced from the C-terminal end of the peptide by coupling a protected amino acid to a suitable resin. A starting material can be prepared by attaching an amino-protected amino acid via a benzyl ester linkage to a chloromethylated resin or a hydroxymethyl resin or via an amide bond to a benzhydrylamine (BHA) resin, a methylbenzhydrylamine (MBHA) resin or a benzyloxybenzyl alcohol resin. These resins are available commercially, and their preparation and use are well known.

General methods for protecting and for removing protecting groups from amino acids which can be used in this invention are described in "The Peptides: Analysis, Synthesis, Biology", Vol. 2, (E. Gross and J. Meienhofer, Eds., Academic Press, New York, p. 1-284 [1979]) and by Atherton et al., in "The Peptides: Analysis, Synthesis, Biology" p. 1-38, Vol. 9, (S. Udenfried and J. Meienhofer, Eds., Academic Press, New York [1987]). Protecting groups include, e.g., the 9-fluorenylmethyloxycarbonyl (Fmoc), tert.-butyloxycarbonyl (Boc), benzyl (Bzl), t-butyl (But), 2-chlorobenzyloxycarbonyl (2Cl-Z), dichlorobenzyl (Dcb) and 3,4-dimethylbenzyl (Dmb) groups.

After removal of the a-amino protecting group from the initial (C-terminal) amino acid, the remaining

protected amino acids are coupled step-wise in the desired order. The entire peptide may be synthesized in this way. Alternatively, small polypeptides may be constructed which are later joined, to give the final peptide product. Appropriate coupling procedures are known in the art, with the procedure of König et al. (Chem. Ber. 103, 788-798 [1970]) using 1,3-dicyclohexylcarbodiimide/1-hydroxybenzotriazole (DCC/HOBt) or the procedures of Dourtoglou et al. (Synthesis 1984, pp. 572-574) and Knorr et al. (Tetrahedron Letters, 30, 1927-1930 [1989]) using O-benzotriazolyl-N,N,N′,N′-tetramethyluronium hexafluorophosphate (HBTU) being particularly suitable.

Each protected amino acid or peptide is introduced into the solid phase reactor in excess, and the coupling may be carried out in a medium of dimethylformamide (DMF) or methylene chloride ($CH_2Cl_2$), or a mixture thereof. In cases where incomplete coupling occurs, the coupling procedure is repeated before removal of the Nα-amino protecting group prior to the coupling of the next amino acid. The success of the coupling reaction at each stage of synthesis may be monitored. A preferred method of monitoring the synthesis is by the ninhydrin reaction. The coupling reactions and washing steps can be performed using automated instrumentation.

Cleavage of the peptide from the resin can be effected using procedures well known in peptide chemistry. For example, reaction with hydrogen fluoride (HF) in the presence of p-cresol and dimethylsulfide at 0° C for 1 hour may be followed by a second reaction with hydrogen fluoride in the presence of p-cresol for 2 hours at 0° C or with trifluoroacetic acid/methylene chloride/anisole. Cleavage of peptides from chloromethylated or p-benzyloxybenzyl alcohol resin supports produces finished peptides having carboxyl groups at the C-termini. Cleavage of peptides from benzhydrylamine or methylbenzhydrylamine resins produces peptides having C-terminal amide groups.

Alternatively the peptides or polypeptides representing the B-cell epitope can be prepared using methods of the recombinant DNA technology. The methods for preparing polypeptides by recombinant DNA technology are well known in the art. A DNA fragment coding for such a polypeptide may be isolated from a genomic library or from a cDNA library from the pathogenic agent or may be prepared according to procedures well known in the art, e.g. by the phosphotriester method (Narang et al., Meth. Enzymol. 68, 90-98 [1979]) or the phosphodiester method (Brown et al., Meth. Enzymol. 68, 109-151 [1979]. The DNA fragment may then be cloned into an expression vector as described by Maniatis et al. in "Molecular Cloning - A Laboratory Manual", Cold Spring Harbor Laboratory [1982].

A large number of haptens and carbohydrates representing a B cell epitope are known to the person skilled in the art. These haptens and carbohydrates may be prepared by well known methods. Alternatively the compound representing the B cell epitope may be prepared by fragmenting the said pathogenic agent and isolating the said compound by biochemical methods.

The compound representing a T cell epitope is a peptide, a polypeptide or a carbohydrate derived from a pathogenic agent. The said compound may be prepared as indicated above for the compound representing a B cell epitope.

Furthermore the peptide, polypeptide, hapten or carbohydrate representing the B-cell epitope and/or the peptide or polypeptide representing the T-cell epitope may be a multiple antigenic peptide (MAP). Such MAP's may be prepared as described by Posnett et al., J. Biol. Chem. 263, 1719-1725 [1988]. An example of such a MAP is the multiple antigenic peptide system (MAPS) B-cell epitope $[(NANP)_3]_8$-$Lys_7$-Aca-Cys-$NH_2$ comprising multimers of the repeat sequence (NANP) present in the CS protein of Plasmodium falciparum (International Patent Application No. PCT/US85/01416, Publication No. WO 86/00911). This MAPS can be synthesized by a conventional solid phase procedure. Although lysine is the preferred core molecule in the dendritic structure of MAPS other lysine-like molecules such as ornithine and nor-lysine may also be used.

The compositions of the present invention may be prepared by covalently coupling a compound representing a B cell epitope with a compound representing a T cell epitope. The coupling may be either directly by the formation of a peptide or an ester bond between free carboxyl, amino or hydroxyl groups on the peptide or polypeptide or carbohydrate representing the T-cell epitope and corresponding groups on the peptide, polypeptide, hapten or carbohydrate representing the B-cell epitope or indirectly via a conventional bifunctional linking group. Examples for conventional bifunctional linking reagents used for the formation of such linking groups are sulfosuccinimidyl 4-(p-maleimidophenyl)butyrate (sulfo-SMPB), sulfosuccinimidyl(4-iodoacetyl)aminobenzoate (sulfo-SIAB), N-succinimidyl(4-iodoacetyl)aminobenzoate (SIAB), 2-iminothiolane•HCl (Traut's reagent), dimethyl pimelimidate•2HCl (DMP), succinimidyl 4-(p-maleimidophenyl)butyrate (SMPB), N-succinimidyl 3-(2-pyridyldithio)propionate (SPDP), bismaleimidohexane (BMH) and m-maleimidobenzoyl-N-hydroxysuccinimide ester (MBS). These and further bifunctional linking reagents are commercially available from Pierce Chemical Company, Rockford, Illinois, U.S.A. Alternatively $C_{2-7}$-dialkanals such as glutaraldehyde (Avrameas, Immunochem. 6, 43-52 [1969]) may be used.

The compositions of the present invention may also be prepared directly in the form of a linear peptide comprising the B cell epitope function and the T cell epitope function in one molecule e.g. by using methods of the recombinant DNA technology or by conventional peptide synthesis methods. However there is no need that the compound representing the T-cell epitope is covalently linked to the compound representing the B-cell epitope, only that these compounds be associated in such a way as to lead to joint presentation to cells of the immune system.

The compositions and compounds according to the present invention can be purified by known methods, such as differential centrifugation, precipitation with ammonium sulfate, dialysis to remove salts (under normal or reduced pressure), preparative iso-electric focusing, preparative gel electrophoresis or various chromatographical methods, e.g., gel filtration, high performance liquid chromatography (HPLC), ion exchange chromatography, reverse phase chromatography or affinity chromatography.

The present invention also relates to vaccines comprising a composition of the present invention and a pharmaceutically acceptable adjuvant.

The said vaccine can be used to induce a protective immune response against a pathogenic agent having a B-cell epitope immunologically cross-reactive with the one in the composition. The term "pharmaceutically acceptable adjuvant" can mean either the standard compositions which are suitable for human administration e.g. aluminum hydroxide or the typical adjuvants and excipients (e.g. serum albumin or plasma preparations) employed in animal vaccinations. Suitable adjuvants for the vaccination of animals include but are not limited to Freund's complete or incomplete adjuvant (not suitable for human or livestock use), Adjuvant 65 (containing peanut oil, mannide monooleate and aluminum monostearate), mineral gels such as aluminum hydroxide, aluminum phosphate and alum, surfactants such as hexadecylamine, octadecylamine, lysolecithin, dimethyl-dioctyldecylammonium bromide, N,N-dioctadecyl-N′,N′-bis(2-hydroxyethyl)propanediamine, methoxyhexydecylglycerol and pluronic polyols, polyanions such as pyran, dextran sulfate, polyIC, polyacrylic acid and carbopol, peptides and amino acids such as muramyl dipeptide, dimethylglycine, tuftsin and oil emulsions. The composition of the present invention can also be administered following incorporation into liposomes or other micro-carriers, or after conjugation to polysaccharides, other proteins or other polymers or in combination with Quil-A to form "Iscoms" (immunostimulating complexes) (Allison et al., J. Immunol. Meth. 95, 157-168 [1986]; Morein et al., Nature 308, 457-460 [1984]). In addition, genetically engineered microorganisms such as vaccinia or salmonella which are capable of expressing a nucleotide sequence encoding a peptide or polypeptide representing a T-cell epitope and a B-cell epitope i.e. a composition according to the present invention can be used as vaccine delivery systems (Mackett, Immunol. Letters 16, 243-248 [1987]).

The vaccines are prepared by combining a composition according to the present invention with a pharmaceutically acceptable adjuvant. Preferably the vaccine is in the form of a unit dose. The amount of active compounds administered as a vaccination or as a medicament at one time, or over a period of time, will depend on the subject being treated, the manner and form of administration, and the judgement of the treating physician. However, an effective dose may be in the range of from about 1 ng to about 1 mg of the composition of this invention, preferably about 100 µg to about 500 µg; it being recognised that lower and higher doses may also be useful. The vaccine may be in a variety of forms. These include, for example solid, semi-solid and liquid dosage forms. The unit dose is preferably packed in 1 ml vials containing the vaccine in the form of a suspension in sterile 0.9% (w/v) NaCl solution. The most preferred vaccine comprises 0.4 mg/ml protein (T and B cell epitope peptides) adsorbed to 850 µg $Al(OH)_3$/ml and 100 µg/ml Merthiolate™ (Eli Lilly). The vial is preferably packed in a container together with written instructions describing the correct use of the vaccine. The present invention relates also to such a unit dose of the vaccine packed in a container, most preferably together with the appropriate instructions. Furthermore the present invention relates to a process for the preparation of said vaccines or of a unit dose thereof as well as to a method for the immunization of a human or animal using such an vaccine.

The form and the route of administration of a vaccine as well as frequency of injections are all factors which can be optimized using ordinary skill in the art. Typically, the initial vaccination with an immunologically effective amount of a vaccine is followed some weeks later by one or more "booster" vaccinations, the net effect of which is the production of high titers of antibodies against the particular pathogenic agent.

Having now generally described this invention, the same may be more readily understood by reference to the following example. It should be understood that this example is for illustrative purposes only and should not be construed as limiting this invention in any way to the specific embodiment recited therein.

## Example

Selection of compounds representing T cell epitopes from TT

The compounds representing T helper cell sequences from TT were obtained by chemical and enzymatic treatment of TT. 50 mg TT were reduced in 5.5 ml 4 M guanidine, 0.4 M Tris buffer pH 8 and 15 mg dithiothreitol. The mixture was incubated for 1.5 hours at room temperature. Then the mixture was alkylated by adding 60 mg iodoacetamide and incubating for 0.5 hours at room temperature. After an overnight dialysis against 0.1 M $NH_4HCO_3$ adjusted to pH 7 with glacial acetic acid, 1.8 mg trypsin (2 U/mg) were added and the mixture was incubated at 37°C overnight. The peptides in the resulting digest were separated after dialysis against 100 mM ammonium carbonate pH 7 using gel filtration FPLC (Sepharose™ 12 column, HR16/50, Pharmacia). Fractions from the column were monitored for activity using T cell proliferation assays (see below) with peripheral blood leukocytes from human donors immunized with TT or lymphocytes from mice immunized with TT. Active fractions were further separated by diluting them 1:1 in trifluoroacetic acid (TFA) and applying them to a reversed phase FPLC column (PepRPC 5/2, Pharmacia) equilibrated with 0.1% TFA. Peptides were eluted with a linear gradient of isopropanol. Again, aliquots were tested for activity in an in vitro T cell proliferation assay. Active aliquots were further separated, after 1:1 dilution in TFA, on a reversed phase HPLC column (Vydac $C_{18}$ No. 218TP544) equilibrated with 0.1% TFA. Peptides were eluted with a linear gradient of acetonitril. Fractions were tested for activity with the in vitro T cell proliferation assay. Active fractions were characterized by N-terminal sequencing and amino acid analysis. Once characterized, one of the peptides, i.e. TT73-99 and derivatives thereof such as TT88-99 were synthesized and tested for activity in T cell proliferation tests, helper T cell tests and in epitope suppression tests as described below for the exemplary composition $(NANP)_4$TT73-99.

Synthesis and purification of $(NANP)_4$TT73-99 and TT73-99

The polypeptide TT73-99 having the amino acid sequence (I) corresponds to the amino acid residues 73-99 of the tetanus toxoid. The polypeptide TT73-99 was synthesized by the solid-phase technique using base-labile N-fluorenylmethoxylcarbonyl-amino acids, t-butyl based side chain protecting groups and a p-benzyloxybenzylalcohol polystyrene resin as described by Atherton and Sheppard in "The Peptides: Analysis, Synthesis, Biology" p. 1-38, Vol. 9, S. Udenfriend and J. Meienhofer (Eds.), Academic Press, New York (1987). The initial synthesis was started with the Fmoc-Lys(Boc)-O-$CH_2C_6H_4O$-$CH_2C_6H_4$-resin in a manual shaker. The protocol for a typical synthetic cycle was as follows:

| Step | Reagent | Time |
|---|---|---|
| 1 | N,N-dimethylformamide (DMF) | 2 x 1 min. |
| 2 | 20% piperidine/DMF | 1 x 7 min. |
| 3 | DMF | 5 x 1 min. |
| 4 | 2,5 eq. Fmoc-amino acid / DMF<br>+ 2,5 eq. HBTU<br>+ 2,5 eq. N-ethyldiisopropylamine | 1 x 90 min. |
| 5 | DMF | 3 x 1 min. |
| 6 | isopropyl alcohol (i-PrOH) | 2 x 1 min. |

The resulting protected TT73-99 polypeptide resin was treated with trifluoroacetic acid-methylene chloride-anisol (49:49:2) to yield the free TT73-99 polypeptide. This polypeptide was purified by high-performance liquid chromatography (HPLC) using a Lichrosorb RP18 (10$\mu$) column (Merck, Darmstadt, FRG) in a 0,1% trifluoroacetic acid-ethanol gradient system. The polypeptide was homogeneous by analytical HPLC and showed the expected amino acid composition after acid hydrolysis.

The polypeptide $(NANP)_4$TT73-99 was synthesized by a combination of the classical solution technique and solid phase peptide synthesis. The protected tetrapeptide Fmoc-Asn-Ala-Asn-Pro-OH was synthesized according to the following scheme:

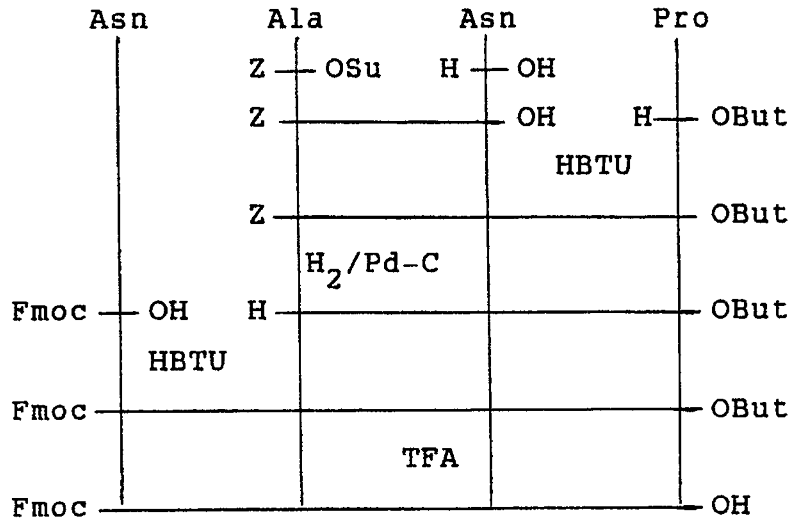


Four repeated couplings of the $N^\alpha$-protected tetrapeptide via the HBTU procedure to the $N^\alpha$-unprotected TT73-99 polypeptide resin yielded the protected $(NANP)_4$TT73-99 polypeptide resin. Treatment with trifluoroacetic acid (TFA)/methylene chloride/anisole liberated the free polypeptide. Purification was achieved by HPLC in the above mentioned gradient system. The polypeptide was homogeneous by analytical HPLC.

Test for activity of $(NANP)_4$TT73-99

As a compound representing a T cell epitope the polypeptide TT73-99 was tested as a model carrier peptide for the B cell epitope sequence $(Asn\text{-}Ala\text{-}Asn\text{-}Pro)_n$ also termed $(NANP)_n$ in the single letter code for amino acids. The repeat sequence (NANP) of the circumsporozoite (CS) protein represents the immunodominant sequence of the major surface protein of P. falciparum sporozoites (Dame et al., Science 225, 593-599 [1984]; Zavala et al., Science 228, 1436-1440 [1985]). Clinical studies with vaccines comprising this sequence have conferred sterile immunity to some of the volunteers challenged with the parasite (Herrington et al., supra; Ballou et al., Lancet i 1277-1281 [1987]). As a compound representing a B cell epitope the peptide $(NANP)_4$ was selected in the present Example.

In the first experiment 5 BALB/c mice were preimmunized by subcutaneous injection with 15 µg TT73-99 in Freund's incomplete adjuvant (FIA). Fifty four days later the preimmunized mice were injected subcutaneously with 25 µg $(Ac\text{-}Cys\text{-}(NANP)_3)_{35}$TT in FIA. The composition $(Ac\text{-}Cys\text{-}(NANP)_3)_{35}$TT which stands for $(Acetyl\text{-}Cys\text{-}(AsnAlaAsnPro)_3]_{35}$-tetanus toxoid was prepared in accordance with known procedures (Etlinger et al., Immunology 64, 551-558 [1988]). At the same day 5 normal BALC/c mice each (not preimmunized with TT73-99) were injected subcutaneously either with 25 µg $(Ac\text{-}Cys\text{-}(NANP)_3)_{35}$TT in FIA or with 25 µg TT in FIA. The antibody titers in the plasma of the mice were measured by ELISA (Etlinger et al., Immunology 64, 551-558 [1988]) between day 54 and day 70 at weekly intervals after the preimmunization using microtiter plates coated with $(NANP)_{50}$, TT or TT73-99. Table II shows the peak response for each immunization protocol. Values are given as geometric mean titers (coefficient variation geometric mean) whereby the titer is defined as the reciprocal of the last dilution of plasma where the optical density at a wavelength of 455 nm ($OD_{455}$) was ≧ 0.1 and ≦ 0.24.

Table II

| Immunization protocol (Injection) | | Antibody titer x $10^3$ | | |
|---|---|---|---|---|
| 1st | 2nd | $(NANP)_{50}$ | TT | TT73-99 |
| - | $(Ac\text{-}Cys\text{-}(NANP)_3)_{35}$TT | 1.4(1.4) | 1.9(1.2) | 0.15(0) |
| TT73-99 | $(Ac\text{-}Cys\text{-}(NANP)_3)_{35}$TT | 18(1.4) | 26(1.5) | 0.54(2) |
| - | TT | 0.15( 0) | 51(1.2) | 0.15(0) |

Table II shows that TT73-99 is recognized by T helper cells since the preimmunization with TT73-99 leads to enhanced anti-(NANP) and anti-TT antibody responses when challenged with the conjugate, i.e. $(Ac\text{-}Cys\text{-}(NANP)_3)_{35}TT$. The absence of antibody cross-reactivity between TT73-99, $(NANP)_{50}$ and TT, as well as the use of a mouse strain which is genetically unresponsive to NANP at the T cell level (Good et al., J. Exp. Med. 160, 655-660 [1986]; Del Guidice et al., J. Immunol. 137, 2952-2955 [1986]) indicates that priming occurred at the helper T cell level.

In the second experiment 2 sets of 5 BALB/c mice were preimmunized by subcutaneous injection with 50 µg TT in $Al(OH)_3$. Thirty six and 168 days later those mice and 2 sets of 5 normal mice were injected subcutaneously either with 25 µg of $(Ac\text{-}Cys\text{-}(NANP)_3)_{35}TT$ or with $(NANP)_4TT73\text{-}99$ in FIA. The antibody titers were measured by ELISA between day 36 and day 70 for the primary peak response and from day 168 and day 189 at weekly intervals for the secondary response. Table III shows the peak response for each immunization protocol. Values are given as geometric mean titers as in Table II.

Table III

| Immunization protocol (Injection) | | Antibody titer x $10^3$ | | |
|---|---|---|---|---|
| 1st | 2nd | $(NANP)_{50}$ | TT | TT73-99 |
| - | $(NANP)_4TT73\text{-}99$ | 3.4(1.5) | 0.37(1.1) | 1.1(1.9) |
| TT | $(NANP)_4TT73\text{-}99$ | 15(1.7) | 98(1.2) | 4.1(1.5) |
| - | $(Ac\text{-}Cys\text{-}(NANP)_3)_{35}TT$ | 21(1.1) | 151(1.2) | 0.15( 0) |
| TT | $(Ac\text{-}Cys\text{-}(NANP)_3)_{35}TT$ | 0.9(1.9) | 454(1.2) | 0.15( 0) |

Table IV

| Immunization protocol (Injection) | | Antibody titer x $10^3$ | | |
|---|---|---|---|---|
| 1st | 2nd and 3rd | $(NANP)_{50}$ | TT | TT73-99 |
| - | $(NANP)_4TT73\text{-}99$ | 116(1.3) | 0.15( 0) | 1.3(1.8) |
| TT | $(NANP)_4TT73\text{-}99$ | 422(1.2) | 173(1.2) | 2.3(1.7) |
| - | $(Ac\text{-}Cys\text{-}(NANP)_3)_{35}TT$ | 179(1.3) | 878(1.3) | 0.15( 0) |
| TT | $(Ac\text{-}Cys\text{-}(NANP)_3)_{35}TT$ | 10(1.3) | 878(1.4) | 0.15( 0) |

Tables III and IV show the comparison of the effect of preimmunization with TT on the subsequent response to the conjugate $(Ac\text{-}Cys\text{-}(NANP)_3)_{35}TT$ containing the entire carrier protein TT and to the peptide $(NANP)_4TT73\text{-}99$ containing only a sub-part of the carrier protein TT viz. TT73-99. A mouse strain which is genetically unresponsive to NANP at the T cell level was used for these studies. As it is shown in Table III for a single challenge with the (NANP)-containing antigen and in Table IV for two challenges with the (NANP)-containing antigen, TT priming inhibited the anti-(NANP) response to $(Ac\text{-}Cys\text{-}(NANP)_3)_{35}TT$ (= epitope suppression) even though the anti-TT response was elevated in pretreated mice. On the other hand TT priming did not lead to an inhibition of the anti-(NANP) reponse to $(NANP)_4TT73\text{-}99$, it actually resulted in the enhancement of the anti-(NANP) reponse after a preimmunization with TT.

The above results demonstrate that epitope suppression here caused by the preimmunization with TT, can be circumvented by supplying carrier information in the form of a peptide derived from the original protein (TT) which peptide (here TT73-99) does not contain information for an epitope suppressive function.

From a practical standpoint, a carrier sequence for vaccines should be recognized by T cells of most people. It is known that certain peptides are restricted by a large variety of human Class II alleles (Sinigaglia et al., Nature 336, 778-780 [1988]; Panina et al., Cold Spring Harbor Symp. Quant. Biol. Vol. LIV, Cold Spring Harbor Laboratory, New York, U.S.A. [1989]). To determine whether TT73-99 could serve as a generally recognized carrier, 2 or 3 mice each of the strains indicated in Table V were injected subcutaneously with $(NANP)_4TT73\text{-}99$ in Freund's complete adjuvant. 32 days later the mice were injected

with 25 µg $(NANP)_4$TT73-99 in FIA (booster). The antibody responses were assayed at weekly intervals for 3 weeks following the booster. Table V shows the peak geometric mean titers of the antibodies formed. Anti-(NANP) titers were determined as described in Table II. Anti-sporozoite titers were determined by indirect immunofluorescence as described by Etlinger et al., J. Immunol. 140, 626-633 [1988]. Normal mouse sera had anti-(NANP) titers ≦ 150 and anti-sporozoite titers < 10. Although the responses were variable, Table V shows that each of the seven mouse strains tested produced anti-(NANP) and anti-sporozoite antibody. Since only H-$2^b$ mice are responsive to the NANP sequence at the T cell level (Good et al., J. Exp. Med. 160, 655-660 [1986]; Del Guidice et al., J. Immunol. 137, 2952-2955 [1986]) the results shown in Table V indicate that all non-H-$2^b$ strains are capable of recognizing the T helper cell epitope represented by TT73-99. In the case of H-$2^b$ mice the helper T cell function could have occured through recognition of the NANP sequence.

Table V

| $(NANP)_4$TT73-99 elicits anti-sporozoite antibody in genetically diverse mice. | | | |
|---|---|---|---|
| Strain | H-2 Haplotype | anti-$(NANP)_{50}$ titer x $10^2$ | anti-sporozoite titer |
| B10.RIII | r | 59 | 28 |
| C57B1/10 | b | 14305 | 115852 |
| B10.s | s | 366 | 1280 |
| B10.BR | k | 915 | 20480 |
| B10.M | f | 9 | 10 |
| B10.G | q | 2288 | 40960 |
| B10.D2 | d | 1448 | 20480 |

Based on the data shown by Sinigaglia et al. (supra) it was suggested that TT73-99 is also generally recognized by T cells of a large number of individual members of the genetically diverse human population. To gain insight into this point, peripheral blood leukocytes (PBL) were obtained from 20 volunteers and tested in an in vitro T cell proliferation assay for reactivity with TT73-99 or TT. PBL were obtained from whole blood. Cultures were set up in duplicate in wells of microtiter plates which had previously received either 20 µl of 100 µg/ml TT73-99 in 0.15 M NaCl or 100 µg/ml TT in 0.15 M NaCl or only 0.15 M NaCl. Each well contained 4 x $10^5$ cells in 0.2 ml of culture medium (RPMI with 10% heat-inactivated human AB serum, 100 units/ml penicillin, 100 µg/ml streptomycin and 2 mM glutamine). The microtiter plates were then incubated at 37° C. The extent of T cell proliferation was determined after 4 days of incubation by adding 1 µCi [$^3$H]-thymidine to each well and determining [$^3$H]-thymidine incorporation after 24 hours. The [$^3$H]-thymidine incorporation is a measure for the extent of DNA synthesis. The stimulation index is the amount of [$^3$H]-thymidine incorporation (measured as counts per minute; abbreviated cpm) in the cultures containing TT73-99 or TT, respectively, divided by the amount [$^3$H]-thymidine incorporation in cultures containing neither TT73-99 nor TT. 12 out of 20 volunteers responded to TT73-99 (stimulation index ≥2) (Table VI). Furthermore, in more limited studies it was found that T cells proliferated in response to $(NANP)_4$TT73-99 but not to $(NANP)_{50}$ or $(NANP)_3$. This indicates that the TT73-99 part can be recognized by T cells i.e. that the presence of the compound representing the B cell epitope (the $(NANP)_4$-part) does not mask the information for the T helper cell function of the compound representing the T cell epitope (the TT73-99 part).

Table VI

| Cellular reactivity to TT and TT73-99 in humans | | | | | | | | |
|---|---|---|---|---|---|---|---|---|
| antigen | Stimulation index / # volunteers | | | | | | | |
| | <2 | 2 | 3 | 4 | 5 | 8 | 14 | 36 |
| TT73-99 | 8 | 3 | 2 | 3 | 1 | 1 | 1 | 1 |
| TT | <2 | 3 | 10-30 | | 40-60 | | 70-100 | >100 |
| | 1 | 1 | 5 | | 5 | | 4 | 4 |

In a further experiment sera from 55 volunteers who had participated in Phase I clinical studies analyzing the adjuvanticity of interferons $\alpha$ and $\gamma$ on responsiveness to $(Ac\text{-}Cys\text{-}(NANP)_3)_{35}TT$ were tested for reactivity with TT and TT73-99 both prior to and following immunization with $(Ac\text{-}Cys\text{-}(NANP)_3)_{35}TT$. Antibody titers were determined as described by Etlinger et al., J. Immunol. 140, 626-633 [1988] using peroxidase-coupled anti-IgM and IgG antibody. Values are geometric mean (coef.var.g.m.) for 55 volunteers. Preclinical titers are those prior to injection with $(Ac\text{-}Cys\text{-}(NANP)_3)_{35}TT$. Geometric mean titer (coef.var.g.m.) of pre- and post-clinical sera in plates coated with bovine serum albumin (used as the blocker in ELISA) was found to be $10^3$ (1.1). Although as mentioned above T cell reactivity to TT73-99 could be demonstrated in about half of the 20 people tested, none of the antisera from the other 55 volunteers reacted with this peptide (Table VII).

Table VII

| Humoral reactivity to TT and TT73-99 in humans | | |
|---|---|---|
| | pre-clinical sera | post-clinical sera |
| | (Titer x $10^3$) | (Titer x $10^3$) |
| TT73-99 | 1.2(1.1) | 1.3(1.1) |
| TT | 44(1.3) | 141(1.2) |

The absence of human antibody reactivity for TT73-99 following TT or $(Ac\text{-}Cys\text{-}(NANP)_3)_{35}TT$ immunization shown above is similar to the results obtained with mice (see Tables III and IV). There it had been shown that the inhibition of the antibody response to the B cell epitope, i.e. the $(NANP)_4$ part of the composition $(NANP)_4$TT73-99, was caused by the preimmunization with the carrier protein TT presumably in part because preimmunization led to the presence of primed, carrier-specific B cells which recognized the unmodified part of the carrier protein TT in the composition. This inhibition has been termed epitope suppression (Schutze et al., Cell. Immunol. 104, 77-90 [1987]). The above results imply that the absence of antibody cross-reactiviiy between the original protein and carrier peptide is a reasonable prerequisite for candidate peptides containing information for carrier function but not for epitope suppressive function.

The next experiment was designed to test the effect of the immunization of a host against a pathogenic agent $P_0$, which host has been preimmunized

(a) first with an antigen from a pathogenic agent $P_1$, e.g. with tetanus toxoid (TT), and then with a composition C, e.g. the composition $(NANP)_4$TT88-99, comprising a compound representing a B cell epitope which is an antigen from a pathogenic agent $P_2$, e.g. an antigen from a malaria parasite, or an antigenic sub-part thereof such as the B cell epitope $(NANP)_4$ from the CS protein of P. falciparum, and a compound representing a T helper cell epitope which is a sub-part of an antigen from the pathogenic agent $P_1$, e.g. from TT, characterized in that the compound representing the T helper cell epitope contains information for carrier function but not for an epitope suppressive function, such as the compound TT88-99 or TT73-99, or

(b) once or twice with the composition C mentioned above,

on the immune response in a host after the immunization with He146-61TT88-99. The titer of the antibodies reactive with the compound representing a B cell epitope He146-61 was determined using a conjugate consisting of Cys-He146-61 coupled to bovine serum albumin (termed He146-61-BSA) prepared according to know procedures (Etlinger et al., Immunology 64, 551-558 [1988]). As shown in Table VIII, part C the antibody response elicited against the B cell epitope He146-61 was enhanced in those mice which had been preimmunized in comparison to non-preimmunized animals. No difference in the effect was seen between the preimmunization protocol (a) vs. (b). Experiment C shows that a preimmunization with a composition of the present invention not only does not result in an inhibition of the immune response against a second composition in accordance with the present invention comprising a compound representing a new B cell epitope and a compound representing the original T cell epitope but actually enhances the response to the new B cell epitope. This means that as new protective B cell epitopes are identified, they can be combined with a previously used T cell epitope such as e.g. TT73-99 or TT88-99 and then be used for the immunization of a host which has been preimmunized with a composition of the present invention comprising a compound representing the said previously used T cell epitope, whereby the immunized host will respond to the said new protective B cell epitope with an enhanced immune response.

In a further experiment the effect of the immunization of a host with a composition of the present invention such as the composition $(NANP)_4$TT73-99, which host has been preimmunized with only the compound representing a T helper cell epitope of the said composition (instead of a preimmunization with the complete antigen wherefrom the T helper cell epitope is derived) has been tested. It was found that this immunization protocol leads to a strong suppression of the immune response against the B cell epitope i.e. $(NANP)_n$ in the composition. It has to be noted however, that the above-mentioned immunization protocol is just of theoretical interest since in practice hosts are not preimmunized with compounds representing T helper cell epitopes only. As clearly indicated above the method for determining whether a compound representing a T helper cell epitope provides carrier function but does not provide an epitope suppressive function clearly involves preimmunizations with the complete antigen and therefore it is understood that the present invention does not relate to cases where the host is preimmunized with the compound representing the T helper cell epitope only but to cases where the host has been preimmunized with the antigen wherefrom the T helper cell epitope is a sub-part thereof or with a composition of the present invention.

The above results demonstrate that it is possible to take advantage of T helper cell priming and eliminate the potential disadvantage of epitope suppression stemming from carrier preimmunization by identifying T helper cell epitopes from currently used protein-containing vaccines and using compounds representing these T cell epitopes alone or in combination with other antigens as carriers for compounds representing B cell epitopes of various pathogenic agents. The present invention provides improved sub-unit vaccines which represent a further step in the development of "engineered" vaccines. These improved sub-unit vaccines are especially important in cases where humoral immunity constitutes an important effector mechanism such as in the prevention of malaria.

## Claims

1. A composition comprising a compound representing a B cell epitope which is an antigen from a pathogenic agent or an antigenic sub-part thereof and a compound representing a T helper cell epitope which is a sub-part of an antigen from a pathogenic agent characterized in that the compound representing the T helper cell epitope contains information for carrier function but not for an epitope suppressive function.

2. A composition according to claim 1 characterized in that the compound representing a T helper cell epitope is a sub-part of an antigen from a pathogenic agent selected from the group of Corynebacterium diphtheriae (Klebs-Loeffler bacillus), Streptococcus pneumoniae, Clostridium tetani, Bacillus Calmette-Guérin, Bordetella pertussis, Vibrio cholerae, mumps virus, measles virus, rubella virus, adenovirus, polio virus, Bacillus anthracis, rota virus, hepatitis virus, rabies virus, HIV-1, HIV-2, HTLV, influenza virus, pox virus, Yersinia pestis, Plasmodium falciparum, foot-and-mouth disease virus, Eimeria tenella and Eimeria acervulina.

3. A composition according to claim 1 characterized in that the compound representing a T helper cell epitope is a sub-part of the tetanus toxin or the diphtheria toxin.

4. A composition according to claim 1 characterized in that the compound representing a T helper cell epitope is the polypeptide having the amino acid sequence

Tyr-Asp-Pro-Asn-Tyr-Leu-Arg-Thr-Asp-Ser-Asp-Lys-Asp-Arg-Phe-Leu-Gln-Thr-Met-Val-Lys-Leu-Phe-Asn-Arg-Ile-Lys, (I)

European Patent Office

# EUROPEAN SEARCH REPORT

Application Number

**EP 90 11 9582**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SCIENCE. vol. 235, 1987, LANCASTER, PA US pages 1059 - 1062; Michael F.et al: "Construction of synthetic immunogen:use of new T-helper epitope...." * the whole document * | 1-2,6-11 | C 07 K 17/02<br>A 61 K 39/385 |
| X,P | EP-A-0 343 460 (F.HOFFMANN-LA ROCHE) * the whole document * | 1-2,6-11 | |
| X,P | EP-A-0 378 881 (ENIRICERCHE) * the whole document * | 1-3,6-11 | |
| X,Y | WO-A-8 906 974 (PRAXIS BIOLOGICS) * the whole document * | 1-3,6-11, 4-5 | |
| Y | EMBO JOURNAL. vol. 5, no. 10, 1986, EYNSHAM, OXFORD GB pages 2495 - 2502; Urlich E. et al: "Tetanus toxin:primary structure,expression..... " * the whole document * | 4-5 | |
| Y | JOURNAL OF IMMUNOLOGY. vol. 142, no. 2, 15 January 1989, BALTIMORE US pages 394 - 402; Stephane D.et al: "Delineation of several DR-restricted tetanus toxin T cell epitopes" * the whole document * | 4-5 | |

TECHNICAL FIELDS SEARCHED (Int. Cl.5)

C 07 K
A 61 K

The present search report has been drawn up for all claims

| Place of search | Date of completion of search | Examiner |
|---|---|---|
| The Hague | 21 February 91 | FERNANDEZ Y BRANAS F |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document